# EUROPEAN PATENT APPLICATION

(11) **EP 4 162 814 A1**
(43) Date of publication of application: **12.04.2023**
(21) Application number: 21818340.8
(22) Date of filing: 24.05.2021
(51) Int. Cl.: A23L 33/105, A23L 19/00, A61K 36/27, A61K 36/53, A61K 36/232, A61P 43/00

(54) **COMPOSITION FOR FATIGUE RECOVERY**

(30) Priority: 05.06.2020 KR 20200068217
(71) Applicant: Natural Endotech Co., Ltd., Gyeonggi-do 13486 (KR)
(72) Inventor: KIM, Jae Soo, Seongnam-si, Gyeonggi-do 13486 (KR); LEE, Yong Wook, Seongnam-si, Gyeonggi-do 13486 (KR); YI, Kwon Taek, Seongnam-si, Gyeonggi-do 13486 (KR)
(74) Representative: Plougmann Vingtoft a/s
(86) International application number: PCT/KR2021/006433
(87) International publication number: WO 2021/246702

(57) **Abstract**

The present invention relates to a composition for fatigue prevention, recovery, or treatment.

## Description

### Technical Field

This patent application claims priority to and the benefit of Korean Patent Application No. 10-2020-0068217 filed with the Korean Intellectual Property Office on June 5, 2020, the disclosure of which is incorporated herein by reference.

The present disclosure relates to a composition for prevention and treatment of fatigue and recovery from fatigue.

### Background Art

Recently, exercise has been expanding beyond competitive sports to the field of preventing diseases and promoting health. Regular exercise makes important contribution to improving health, such as reducing the risk of cardiovascular disease, cancer, osteoporosis, and obesity. In addition, such exercise is known to prolong human lifespan and reduce the risk of premature death.

However, it has been reported that with the increased consumption of oxygen during exercise, a large amount of reactive oxygen species (ROS) is generated, giving rise to the acceleration of oxidative stress and tissue damage in endurance exercises consuming oxygen and provoking an imbalance in endocrine and immune functions. In particular, it has been known that rapid high-intensity exercise can act as a factor that changes circulating hormone concentrations more significantly than mental stress.

Major causes of muscle fatigue occurring during exercise include lack of energy stored in the body, difficulty in transmitting nerve impulses, accumulation of metabolites generated in muscles and blood during exercise. Among the metabolites produced during exercise, lactate is one of the factors for peripheral fatigue, and is widely used as an indicator to determine the energy source of muscles or the degree of fatigue in sports fields.

Particularly when exercising for a long period of time, oxygen deficiency in muscles and acidification of the body due to an increase in lactic acid concentration cause peripheral fatigue. The oxygen deficiency and acidification also increase the toxicity associated with central fatigue and is reported to affect the secretion of neurotransmitters such as dopamine and serotonin in the brain. In addition, there are various indices to determine the energy source of the muscle or the degree of fatigue.

Non-esterified fatty acids are molecules released from triglycerides by the action of the enzyme lipase and are transported in association with serum albumin. Non-esterified fatty acids account for only 5% of total fatty acids, but they are converted quickly and act as an important energy source for peripheral tissues. When the available energy is insufficient due to the decrease in glycogen in the muscle, non-esterified fatty acids are introduced to help muscle activity by supplying energy.

In addition, excessive exercise performance decomposes glycogen in the muscle and converts it into energy, with the consequent breakdown of muscle glycogen. However, when exercise capacity improves, the body increases the content of glycogen in the muscle, reducing fatigue caused by exercise.

Lactate dehydrogenase catalyzes the conversion of lactate to pyruvate and back, as it converts NAD+ to NADH and back. Lactate dehydrogenase is widely present in body tissues such as blood cells and heart muscle, and is used as an important marker of tissue damage because it is released into the bloodstream when tissue damage occurs. In case of vigorous exercise, excessive pyruvic acid is produced, promoting lactic acid formation and increasing the activity of lactate dehydrogenase, which catalyzes the process of converting pyruvic acid into lactic acid.

Enzymatic activities of superoxide dismutase (SOD), catalase (CAT), and glutathione S-transferase (GST) can be used as indices for measuring the activity of antioxidant enzymes in liver tissues.

In addition, glutathione (GSH), an antioxidant, is a major intracellular substance that exhibits non-enzymatic antioxidant activity and has the function of detoxifying radicals in the final stage of antioxidant activity. Malondialdehyde (MDA), a mediator of oxidation reactions, acts as one of the markers of oxidative stress.

Other regulatory factors involved in recovery from exercise fatigue include PPAR-γ, UCP-3, CTP-1, and β-HAD. PPAR-γ is a factor that regulates enzymes involved in the oxidation and transport of fatty acids in muscle tissue, and is involved in fatty acid storage and glucose metabolism. UCP-3 is involved in energy metabolism in muscle cells and inhibits the accumulation of reactive oxygen species (ROS). CTP-1 and β-HAD are factors that act in the regulatory step of mitochondrial migration in fatty acid beta-oxidation.

When the substrates used in the muscles are inappropriate due to muscle dysfunction, fatigue increases and also affects mentally. In order to recover from such fatigue, supply of sufficient energy sources, rest, and suppression and removal of fatigue substances in the body, etc. are required, but in fact, it is difficult to take sufficient nutrition and rest in busy modern social life. Therefore, it is necessary to develop a material that can suppress fatigue or facilitate recovery from fatigue.

In recent years, the demand of general consumers on natural products has been raised as their reluctance to chemically synthesized drugs increases. Therefore, there is an urgent need to develop a composition using natural products for effective prevention or treatment of fatigue or for effective recovery from fatigue.

### Detailed Description of the Invention

### Technical Problem

Accordingly, the present inventors have tried to find a natural product that is excellent for stress suppression, and as a result, through analysis of muscle and liver tissues obtained through animal model experiments, a composition effective for preventing or treating fatigue or facilitating recovery from fatigue was identified.

An aspect of the present disclosure is to provide a food composition for prevention of or recovery from fatigue.

Another aspect of the present disclosure is to a pharmaceutical composition for prevention or treatment of fatigue.

### Technical Solution

The present disclosure pertains to a composition for prevention or treatment of or recovery from fatigue.

Below, a detailed description will be given of the present disclosure.

An aspect of the present disclosure is drawn to a food composition including an extract from at least one selected from the group consisting of Cynanchi Wilfordii Radix, Phlomis umbrosa TURCZ. and an Angelica sp. for prevention of or recovery from fatigue.

In the present disclosure, the fatigue may be central nervous system fatigue, nerve-muscular joint fatigue, peripheral fatigue of the limbs, but is not limited thereto.

In the present invention, fatigue may be accompanied by decreased exercise performance, chronic fatigue, sleep disorder, mental concentration disorder, muscle pain, arthralgia, headache, sore throat or lymphadenitis, but is not limited thereto.

Cynanchi Wilfordii Radix originates from the plant Cynanchum wilfordii Hemsley, which is a vine plant growing in sunny meadows at the foot of mountains or on the slopes of the seaside. The roots of the plant are harvested from fall to winter, dried, and then used as a medicine. Cynanchi Wilfordii Radix is sweet and slightly bitter in taste and has a slightly warm property. In the sense of oriental medicine, this medicine replenishes the energy of the liver and kidneys, strengthens muscles and bones, improves digestion, and has the function of detoxification. Taking advantage of these effects, Cynanchi Wilfordii Radix is applied to the treatment of liver and kidney deficiency syndrome, impotence syndrome, nocturnal emission, soreness of the waist and knees, spleen deficiency, abdominal fullness, diarrhea, early gray hairs compared to age, and insufficient milk secretion after childbirth.

Phlomis umbrosa TURCZ., which is a perennial herb belonging to the Lamiaceae family, is used as an alternative to the dried root of *Dipsacus japonicus* Miq. Due to the rarity thereof. The Chinese name of Phlomis umbrosa TURCZ. was given because it treats fractures well. Its components include alkaloids, essential oils, vitamin E, etc. The plant is mild in medicinal property and bitter in taste. This medicinal material is effective for the treatment of low back pain caused by hepatic or renal dysfunction and the poor skeletal and muscular movement of the legs. Phlomis umbrosa TURCZ. is also therapeutically effective for arthritis and rheumatoid arthritis and is often applied to the treatment of hernia of intervertebral discs and bruises in the back. For women, it can prevent miscarriage when administered during pregnancy, and has the effect of stopping bleeding even when menstruation is excessive or uterine bleeding is severe. For the elderly, it is often used for numbness and pain due to gait disorders due to lack of strength in the lower body or poor flexion and extension. Phlomis umbrosa TURCZ. is recommended to avoid simultaneous use with Rehmannia glutinosa var. purpurea and is not used for treating dysentery. A typical prescription formulation is a pill of Phlomis umbrosa TURCZ. Its gemmules are eaten as a vegetable food.

As used herein, the term "angelica" refers to a dried root from Angelica gigas Nakai in Korea, Angelica sinensis (Oliv.) Diels in China, and Angelica acutiloba (Siebold. & Zucc.) Kitag. or Angelica acutiloba (Siebold. & Zucc.) Kitag. var. sugiyamae Hikino in Japanese. It is said that the Chinese character of angelica was given with the meaning of wanting to return. This name meaning originated from a custom long ago in China where wives worried about their husbands going to the battlefield and put angelica in their clothes because it was believed that when energy was exhausted in the battlefield, the intake of angelica would restore energy and thus the husbands could return back to their wives. According to one theory, it is also named after saying that if this medicine is taken, the vigorous energy will return to the original state. This medicine is warm in vigorous property and sweet and spicy in taste. In general, Angelica gigas Nakai is weaker in sweet taste and spicier than Angelica sinensis (Oliv.) Diels or Angelica acutiloba (Siebold. & Zucc.) Kitag. Angelica's efficacy resorts mainly to blood supplementation, which aid to produce blood when blood is scarce. The roots from Angelica sinensis (Oliv.) Diels or Angelica acutiloba (Siebold. & Zucc.) Kitag are superb in blood supplementation. However, the roots from Angelica gigas Nakai are high in blood circulating activity rather than blood supplementation and exhibits strong anticancer and antihypertensive effects. Pharmaceutically, angelica promotes the blood flow in the coronary artery and accelerates red blood cells. Angelica is given various names in Korea, China, and Japan, such as to-dang-gwi, soong-geom-cho, or Jo-seon-dang-gwi in Korea, Da̅nggul̅, wengu-I, Qián gui, Dà qín, Xiàng mǎ, or de xia̅n yuan in China, and Ni~Tsu to̅ki in Japan.

In the present disclosure, the composition may include an extract from at least one selected from the group consisting of Cynanchi Wilfordii Radix, Phlomis umbrosa TURCZ., and angelica, for example, an extract from a mixture of Cynanchi Wilfordii Radix, Phlomis umbrosa TURCZ. and angelica, or a mixture of a Cynanchi Wilfordii Radix extract, a Phlomis umbrosa TURCZ. extract, and an angelica extract.

In the present disclosure, the Cynanchi Wilfordii Radix extract may be an extract prepared using at least one selected from roots, stems, and leaves of the Cynanchi Wilfordii Radix, for example, an extract from the roots, but with no limitations thereto.

In the present disclosure, the Phlomis umbrosa TURCZ. extract may be an extract prepared using at least one selected from roots, stems, and leaves of the Phlomis umbrosa TURCZ., for example, an extract from the roots, but with no limitations thereto.

In the present disclosure, the angelica extract may be an extract prepared using at least one selected from roots, stems, and leaves of the Phlomis umbrosa TURCZ., for example, an extract from the roots, but with no limitations thereto.

The extract of the present disclosure may be a crude extract obtained by extraction with at least one solvent selected from the group consisting of water and a straight or branched alcohol of 1 to 4 carbon atoms, for example, a crude extract prepared using water as a solvent.

When used as a solvent for preparing a crude extract of the present disclosure, a mixture of water and alcohol may be an aqueous solution containing a straight or branched alcohol of 1 to 4 carbon atoms in an amount of 10 % (v/v) to 100 % (v/v) (exclusive), 20 % to 100 % (v/v) (exclusive), 30 % to 100 % (v/v) (exclusive), 40 % to 100 % (v/v) (exclusive), 50 % to 100 % (v/v) (exclusive), 60 % to 100 % (v/v) (exclusive), or 70 % to 100 % (v/v) (exclusive).

In the present disclosure, the aqueous alcohol solution may be at least one selected from the group consisting of an aqueous methanol solution, an aqueous ethanol solution, an aqueous propanol solution, and an aqueous butanol solution, but is not limited thereto.

In the present disclosure, the content of the extract as an active ingredient in the composition may be appropriately adjusted depending on the type and purpose of use, the patient's condition, the type and severity of symptoms, etc. and may be 0.001 to 99.9 % by weight or 0.1 to 99.9 % by weight and preferably 0.1 to 50 % by weight or 0.1 to 40 % by weight, based on the weight of the solid content, with no limitations thereto.

Hereinafter, a process for preparation of the extract according to the present disclosure will be described in greater detail.

At least one selected from the group consisting of Cynanchi Wilfordii Radix, Phlomis umbrosa TURCZ. and angelica is sectioned, washed with water to remove foreign substances, dried, and then subjected to reflux extraction with an extraction solvent. In this regard, the extraction solvent may be used in an amount of 5- to 20-fold volumes of the weight of the at least one selected from the group consisting of Cynanchi Wilfordii Radix, Phlomis umbrosa TURCZ. and angelica and preferably in an amount of 7- to 15-fold volumes. The extraction was followed by filtration. The filtrate was collected. No particular limitations are imparted to the extraction temperature, but the extraction is conducted at a temperature of 40 to 110°C and preferably at temperature of 55 to 105°C.

The extraction process may be performed once or many times. In a particular embodiment of the present disclosure, re-extraction may be performed after primary extraction. In the case of mass production of herb medicinal extracts, a loss occurs due to the high water content of the herb medicine itself even if effective filtration is conducted. Thus, only low extraction efficiency is obtained after primary extraction. The re-extraction is to prevent the low extraction efficiency. In addition, as a result of examining the extraction efficiency at each stage, it was found that the extract obtained until secondary extraction amounts to 80 to 90% of the total extract.

In an embodiment of the present disclosure, when the extraction process is repeated twice, the residue obtained after primary extraction is subjected to reflux extraction with about 5 to 15 volumes of an extraction solvent and preferably with 8 to 12 volumes of an extraction solvent. Following extraction, filtration was performed. The filtrate was pooled together with that obtained previously and the pool was concentrated in a vacuum to afford an extract as a concentrate.

Although the extraction efficiency can be increased by mixing the filtrates obtained after two rounds of extraction, the extract of the present disclosure is not limited by the number of extractions.

If too small an amount of the solvent is used in preparing the extract of the present disclosure, it is difficult to stir the solution and the solubility of the extract decreases, resulting in a decrease in extraction efficiency. When the solvent is used in an excessively large amount, the amount of solvent treated in the subsequent purification step increases, making it uneconomical and causing problems in handling. Hence, the solvent may be preferably used within the range.

In order to adjust the content of the remaining lower alcohol in the obtained concentrate so as to make it suitable for use as raw material for medicine, the concentrate may be subjected to 1 to 5 rounds and preferably 2 to 3 rounds of azeotropic concentration with about 10 to 30 times, preferably 15 to 25 times, more preferably about 20 times by weight of water, based on the total amount of the concentrate, and equivalent amount of water is added thereto to homogenously suspend the same. The suspension is then lyophilized and/or spray dried to prepare and extract in a powder form.

In the present disclosure, the health functional food may be a food, a beverage, a food additive, or the like.

In the present disclosure, the content of the extract as an active ingredient contained in the health functional food may appropriately vary depending on the form of food, desired use, etc., with no specific limitations thereto. For example, it may be added in an amount of 0.01 to 15 wt % of the total food, and for health beverage composition, it can be added in an amount of 0.02 to 10 g, preferably 0.3 to 1 g, based on 100 ml of the composition.

Provided that the health beverage composition of the present disclosure contains the above-described extract as an essential component at the indicated ratio, no particular limitations are imparted to the other liquid components, wherein the other components may include various flavorants or natural carbohydrates as in conventional beverage.

Examples of the natural carbohydrates include typical sugars, such as monosaccharides, e.g., glucose, fructose, etc., disaccharides, e.g., maltose, sucrose, etc., and polysaccharides, e.g., dextrin, cyclodextrin, etc., and sugar alcohols such as xylitol, sorbitol, erythritol, etc.

In addition to those described above, natural flavorants (taumatin, stevia extract (e.g., levaudioside A, glycyrrhizin, and the like), and synthetic flavorants (saccharin, aspartame, and the like) may be advantageously used. The amount of the natural carbohydrate generally ranges from about 1 to 20 g and preferably from about 5 to 12 g per 100 ml of the composition of the present disclosure.

The composition of the present disclosure may contain are various nutrients, vitamins, minerals (electrolytes), aromatics such as synthetic and natural aromatics, colorants, thickeners, (cheese, chocolate, etc.), pectic acid and a salt thereof, alginic add and a salt thereof, organic acid, protective colloidal thickeners, pH adjusting agents, stabilizers, preservatives, glycerin, alcohols, carbonizing agents used in carbonate beverage, and etc. Further, the composition of the present disclosure may contain fruit flesh for use in preparing natural fruit juice, fruit juice beverages, and vegetable beverages. The ratio of the additives is not so important, but may be generally in the range of about 0 to 20 parts by weight based on 100 parts by weight of the present disclosure.

In particular, when the extract of the present disclosure is administered to the human body, it is considered that there is no concern about side effects compared to other synthetic drugs in view of the general characteristics of natural extracts. In fact, as a result of the toxicity test on the standardized herbal composition, the extract was found to have no toxic effects on the living body.

Another aspect of the present disclosure is drawn to a pharmaceutical composition comprising an extract from at least one selected from the group consisting of Cynanchi Wilfordii Radix, Phlomis umbrosa TURCZ. and angelica for prevention or treatment of fatigue.

In the present disclosure, the fatigue may be central nervous system fatigue, nerve-muscular joint fatigue, peripheral fatigue of the limbs, but is not limited thereto.

In the present invention, fatigue may be accompanied by decreased exercise performance, chronic fatigue, sleep disorder, mental concentration disorder, muscle pain, arthralgia, headache, sore throat, or lymphadenitis, but is not limited thereto.

The extract is the same as that described above, so its description is omitted.

In the present disclosure, the pharmaceutical composition may further include pharmaceutically suitable and physiologically acceptable auxiliary agents such as a carrier, an excipient, and a diluent in addition to the mixed extract.

In the present disclosure, the carrier, excipient, and diluent which may be contained in the composition may exemplified by lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil.

When the composition is formulated, diluents or excipients such as fillers, extenders, binders, wetting agents, disintegrants, surfactants, etc. may be used. A solid formulation for oral administration may include a tablet, a pill, a powder, granules, a capsule, etc. Such solid formulations may be prepared by mixing the strain or the vesicle derived from the strain with at least one excipient, for example, starch, calcium carbonate, sucrose or lactose, gelatin, etc. In addition to the simple excipients, a lubricant, such as magnesium stearate or talc, may be used.

A liquid formulation for oral administration may include a suspension, a solution for internal use, an emulsion, a syrup, etc. In addition to a simple diluent commonly used, such as water and liquid paraffin, the formulation may include various excipients such as a humectant, a sweetener, an aromatic, a preservative, etc.

A formulation for parenteral administration may include a sterilized aqueous solution, a non-aqueous solvent, a suspension, an emulsion, a lyophilized formulation, a suppository, a transdermal agent, etc. The non-aqueous solvent and the suspension may include propylene glycol, polyethylene glycol, a vegetable oil such as olive oil, an injectable ester such as ethyl oleate, etc.

As a base of the suppository, witepsol, macrogol, Tween 61, cocoa butter, laurin butter, glycerogelatin, etc. may be used.

In an embodiment of applying the composition of the present disclosure to humans, the composition of the present disclosure may be administered alone, but generally in combination with a pharmaceutical carrier selected in consideration of administration modes and standard pharmaceutical practice.

By way of example, the pharmaceutical composition may be administered orally, intraorally, or sublingually in the form of a tablet containing starch or lactose, a capsule containing a suitable excipient, or an elixir or suspension containing a flavoring or coloring agent. Such liquid agents may be formulated together with a pharmaceutically acceptable additive such as a suspending agent (e.g., semi-synthetic glycerides such as methyl cellulose and Witepsol, a mixture of apricot kernel oil and PEG-6 ester, or a glyceride mixture of PEG-8 and caprylic/capric glyceride).

The dose of the composition of the present invention may vary depending on patient's age, weight, and sex, a mode of administration, patients' health conditions, and the severity of disease, and it may be administered once to several times as divided a day at certain intervals according to the judgment of doctors or pharmacists. For example, the daily dose may range from 0.1 to 500 mg/kg on the basis of content of the active ingredient. The dosage is an example of average cases and the dosage may be higher or lower according to the difference of individuals.

When the daily dosage of the composition of the present disclosure is below the lower limit of the dose range, a significant effect cannot be obtained. A dose higher than the upper limit is economically disadvantageous. It is recommended to use the composition within the aforementioned range because an amount deviating a usual dose range may be apt to cause undesired side effects.

So long as it is usually available, any extraction method may be applied to the present disclosure. Examples of the extraction method include cold precipitation, hot water extraction, ultrasonic extraction, and reflux cold precipitation, with preference for hot water extraction, but are not limited thereto.

### Advantageous Effects

The present disclosure relates to a composition for prevention or treatment of or recovery from fatigue. The composition according to the present disclosure may be available as a composition useful for preventing or treating fatigue or facilitating recovery from fatigue.

### Brief Description of the Drawings

FIG. 1 is a schematic view illustrating the process of exhaustive swimming test according to an embodiment of the present disclosure.
FIG. 2 is a graph illustrating the influence of the exhaustive swimming test on myokinetics according to an embodiment of the present disclosure.
FIG. 3 is a graph of glycogen contents in tissues as measured according to an embodiment of the present disclosure.
FIG. 4 is a graph of lactate dehydrogenase levels in tissues as measured according to an embodiment of the present disclosure.
FIG. 5a is a graph of PPAR-γ levels as measured by RT-PCR according to an embodiment of the present disclosure.
FIG. 5b is a graph of UCP-3 levels as measured by RT-PCR according to an embodiment of the present disclosure.
FIG. 6 is a graph of activities of catalase, superoxide dismutase, and glutathione S-transferase as measured according to an embodiment of the present disclosure.
FIG. 7a is a graph of glutathione levels as measured according to an embodiment of the present disclosure.
FIG. 7b is a graph of malondialdehyde levels as measured according to an embodiment of the present disclosure.

### Best Mode for Carrying Out the Invention

The present disclosure relates to a food composition including an extract from at least one selected from the group consisting of Cynanchi Wilfordii Radix, Phlomis umbrosa TURCZ., and angelica for prevention of or recovery from fatigue.

### Mode for Carrying Out the Invention

A better understanding of the present disclosure may be obtained through the following examples which are set forth to illustrate, but are not to be construed as limiting the present disclosure.

### EXAMPLE 1. Preparation of Crude Exact

The natural herbal materials *Cynanchum wilfordii* roots, *Phlomis umbrosa* roots, and *Angelica gigas* roots were mixed at a weight ratio of 1:1:1.08 and subjected to extraction by heating in 10 volumes of water at 95 to 105°C for 8 hours. Following filtration, the filtrate thus obtained was lyophilized at -80°C to afford a crude extract as a powder.

### EXPERIMENTAL EXAMPLE 1. Design of Exhaustive Swimming Test Model for Evaluating Degree of Recovery from Fatigue

To assay a degree of recovery from fatigue, an exhaustive swimming test, which is a modification of the loaded forced swimming test of Moriura T. et al., was carried out.

An acrylic-based transparent plastic pool (90×45×45 cm³) was filled up to 38 cm in height with distilled water. A pump was used to create a one-way flow of 7.5 L/min and the temperature was maintained at 34±1°C.

Five-week-old male ICR mice were purchased and acclimatized in the animal room for a week under a certain condition, then acclimatized to the swimming test once a day from three days prior to the experiment until completion of swimming tests (the time point when the noses of the mice were immersed below the surface of water for 5 seconds).

One hour before the experiment, control mice received an AIN-93M diet-based isocaloric diet with either saline (control) while experimental groups were given the composition of the Example at a dose of 50 mg/kg/day or 200 mg/kg/day. The experiment was carried out until completion of swimming (the time point when the noses of the mice were immersed below the surface of water for 5 seconds).

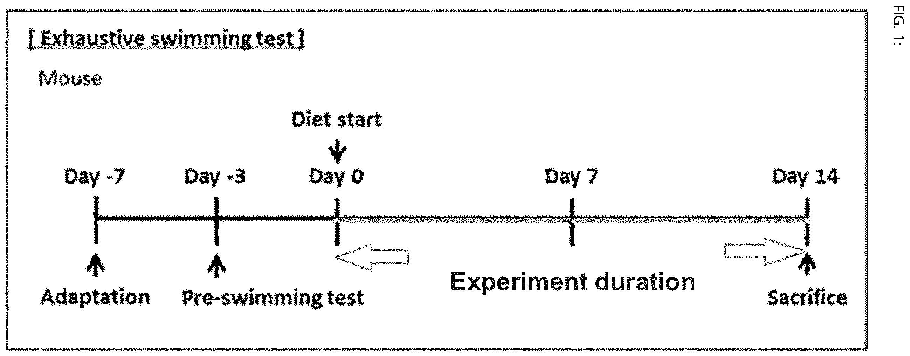

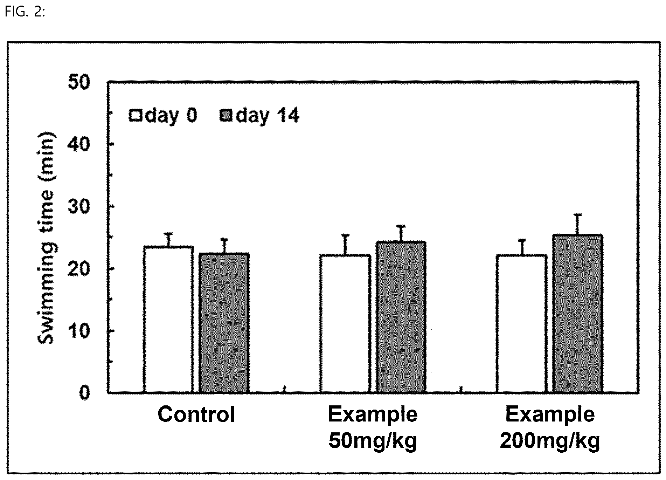

As shown in FIG. 1, the exhaustive swimming test was repeated for two weeks. After termination of the exhaustive swimming test on day 14, examination was made of myokinetics during the exhaustive swimming test and the results are summarized in Table 1 and depicted in FIG. 2.

**TABLE 1**

| | Swimming time (min) | |
|---|---|---|
| | Day 0 | Day 14 |
| Control | 23.46±2.08 | 22.43±2.15 |
| Ex. 1 50 mg/kg | 22.17±3.11 | 24.2712.51 |
| Ex. 2 200 mg/kg | 22.06±2.46 | 25.3613.24 |

As can be seen in FIG. 2, on day 0, the day before treatment, bias caused by mice was minimized by setting the baseline values for reference in further experiments, with the aim of comparatively observing changes during the intake of candidate materials. The swimming time was similar between the control group and the example intake groups. As a result of the measurement on Day 14 after 14 days of experimentation, it was confirmed that there was no change in the swimming time of Day 0 and Day 14 in the control group.

On the other hand, the experimental group ingesting 50 mg/kg of the extract of the Example increased in the swimming time by about 1.09 times when Day 0 and Day 14 were compared. In addition, for the group ingesting 200 mg/kg of the extract of the Example, the swimming time was increased by about 1.15 times when Day 0 and Day 14 were compared. These data demonstrated that the swimming time was increased in a dose-dependent manner.

### EXPERIMENTAL EXAMPLE 2. Evaluation of Intramuscular Glycogen Level and Lactate Dehydrogenase Activity

The exhaustive swimming test mouse models were examined for recovery from fatigue by analyzing their muscle tissues and sera after administration of the control and the extract of the Example for two weeks. After two weeks of the exhaustive swimming test, the mice were sacrificed and muscles were excised from the mice. Intramuscular glycogen levels and blood lactate dehydrogenase (LDH) levels, which both account for muscle fatigue, were measured.

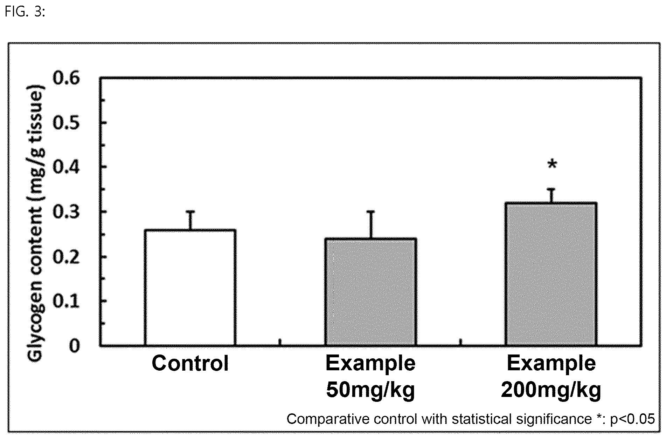

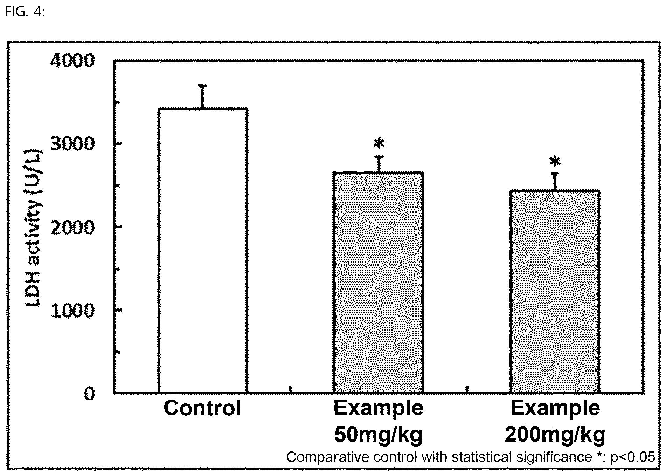

Intramuscular glycogen levels and blood LHD levels were measured by Enzyme-Linked ImmunoSorbent Assay (ELISA) and the measurements are summarized in Table 2 and depicted in FIG. 3 for glycogen levels and in FIG. 4 for LHD levels.

**TABLE 2**

| | Diet | Glycogen (mg/g) | LHD (U/L) |
|---|---|---|---|
| Exhaustive swimming test for 2 weeks | Control | 0.2610.04 | 3,4201275 |
| | Example 50 mg/kg | 0.2410.06 | 2,6511194 * |
| | Example 200 mg/kg | 0.32±0.03 * | 2,4341207 * |

| | | | |
|---|---|---|---|
| Comparative control with statistical significance *: *p<0.05* | | | |

As shown in FIG. 3, the glycogen content in the muscle showed similar values between the control group and the Example 50 mg/kg administration group. On the other hand, the glycogen concentration in the group administered 200 mg/kg of the Example increased by about 1.23 times, compared to the control group, indicating a statistically significant increase.

In addition, as shown in FIG. 4, when comparison was made of the control and the Example groups after two weeks of the exhaustive swimming test, LDH levels showed a significant decrease in both groups in which the examples were administered at low concentration (50 mg/kg) and high concentration (200 mg/kg). In particular, the LDH levels were decreased in a dose-dependent manner by 0.77 times for the 50 mg/kg-administered group and by about 0.71 times for the 200 mg/kg-administered group, compared to the control.

### EXPERIMENTAL EXAMPLE 3. Evaluation of Expression of Regulatory Factors Involved in Recovery from Exercise Fatigue

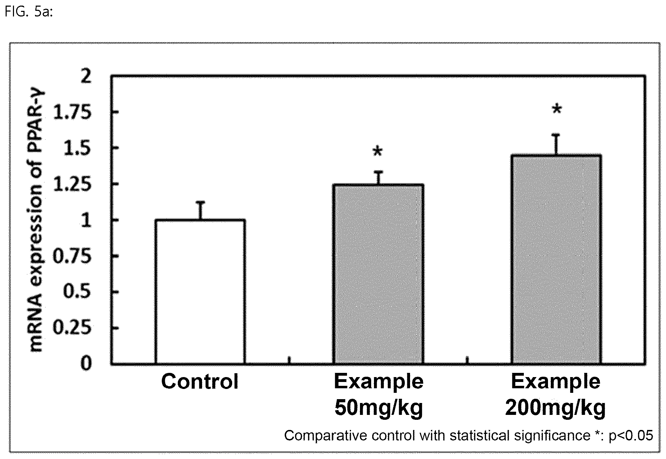

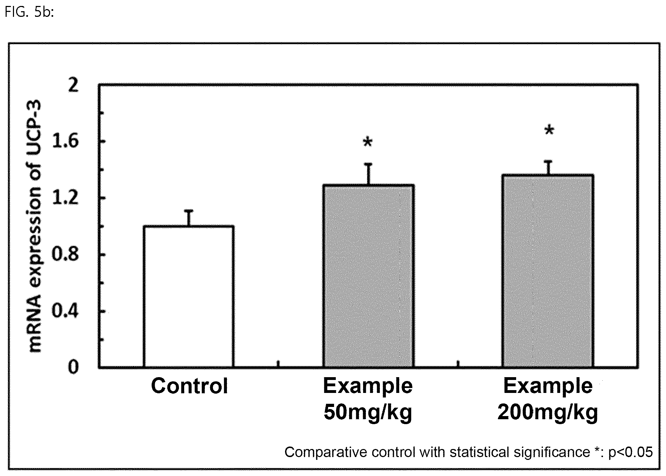

The exhaustive swimming test mouse models were examined for recovery from exercise fatigue by analyzing their muscle tissues for expression of PPAR-γ and UCP-3, which are involved in recovery from exercise fatigue after administration of the control and the extract of the Example for two weeks. mRNA was extracted from muscular tissues and measured for expression levels of PPAR-γ and UCP-3 genes by RT-PCR. Measurements are summarized in Table 3 and depicted in FIG. 5a for PPAR-γ gene expression and in FIG. 5b for UCP-3 gene expression.

**TABLE 3**

| | PPAR-γ | UCP-3 |
|---|---|---|
| Control | 1.0010.12 | 1.0010.11 |
| Example 50 mg/kg | 1.24±0.09 * | 1.29±0.15 * |
| Example 200 mg/kg | 1.45±0.14 * | 1.36±0.10 * |

| | | |
|---|---|---|
| Comparative control with statistical significance *: *p<0.05* | | |

As shown in FIGS. 5a and 5b, the expression of PPAR-γ and UCP-3 in muscle tissue showed a significant increase, compared to the control group, at all concentrations of the Example extract administered. In particular, the PPAR-γ expression level was about 1.24 and 1.45 times higher in the groups to which 50 mg/kg and 200 mg/kg of the Example were administered, respectively, compared to the control group. In addition, the UCP-3 expression level was about 1.29 times and 1.36 times higher in the groups to which 50 mg/kg and 200 mg/kg of the Example were administered, respectively, compared to the control group. These data indicated that the extract of the Example increased the expression levels of PPAR-γ and UCP-3, which are regulatory factors involved in recovery from exercise fatigue, in a dose-dependent manner.

### EXPERIMENTAL EXAMPLE 4. Effect on Antioxidant Enzyme Activity in Vivo

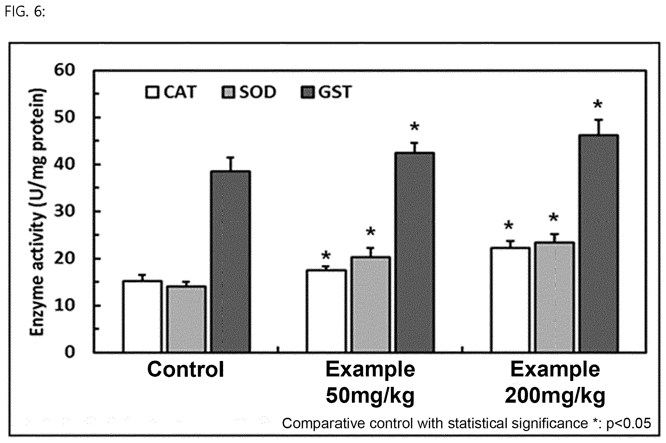

After two weeks of the exhaustive swimming test as in Experimental Example 1, the effect of the extract of the Example on antioxidant enzyme activity was examined. In this regard, the liver tissues from the exhaustive swimming test mouse model were measured for activity of catalase (CAT), superoxide dismutase (SOD), and glutathione S-transferase (GST), and the results are summarized in Table 4 and depicted in FIG. 6.

**TABLE 4**

| | CAT (U/mg protein) | SOD (U/mg protein) | GST (U/mg protein) |
|---|---|---|---|
| Control | 15.28±1.21 | 14.12±0.94 | 38.4113.03 |
| Example 50 mg/kg | 17.54±0.78 * | 20.31±1.98 * | 42.36±2.12 * |
| Example 200 mg/kg | 22.18±1.54 * | 23.40±1.73 * | 46.24±3.25 * |

| | | | |
|---|---|---|---|
| Comparative control with statistical significance *: *p<0.05* | | | |

As seen in Table 4 and FIG. 6, the 50 mg/kg and 200 mg/kg administration groups of the extract of the Example showed significant increases in catalase, superoxide dismutase, and glutathione S-transferase enzyme activities, compared to the control group. In particular, compared to the control group, catalase showed an increase of about 1.14 times and about 1.45 times in the 50 mg/kg- and 200 mg/kg administration groups of the extract of the Example. In the case of superoxide dismutase, the 50 mg/kg and 200 mg/kg administration groups of the extract of the Example showed an increase of about 1.43 times and about 1.65 times, respectively, compared to the control group. Glutathione S-transferase levels increased by about 1.10 times in the 50 mg/kg group and by about 1.20 times in the 200 mg/kg group, compared to the control group. These data indicated that the extract of the Example increased activities of catalase, superoxide dismutase, and glutathione S-transferase, which are indicators of antioxidant enzyme activity in liver tissue in a dose-dependent manner.

### EXPERIMENTAL EXAMPLE 5. Assay for Antioxidant Enzyme Activity in Liver Tissue

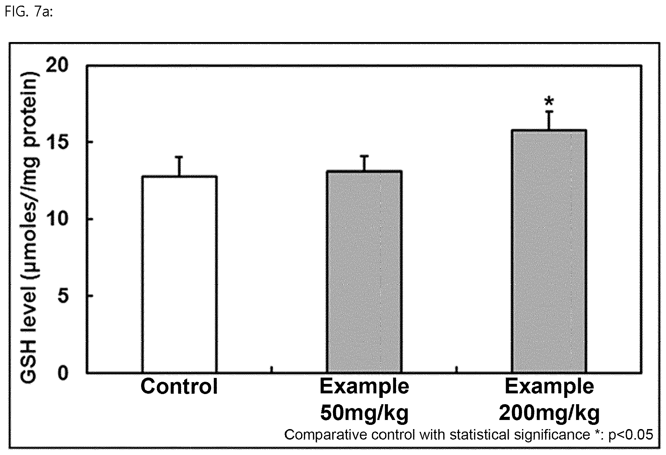

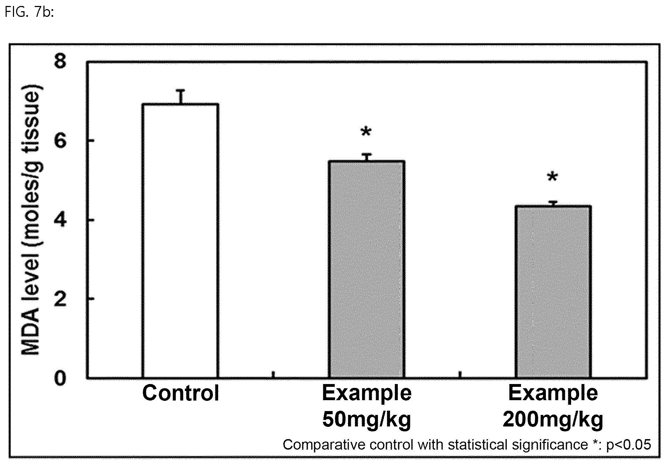

After two weeks of the exhaustive swimming test as in Experimental Example 1, the effect of the extract of the Example on non-enzymatic antioxidant activity and lipid oxidation was examined. In this regard, the liver tissues from the exhaustive swimming test mouse model were measured for levels of the antioxidant material glutathione (GSH) and the tissue lipid oxidation intermediate malondialdehyde (MDA), and the results are summarized in Table 6 and depicted in FIGS. 7a and 7b.

**TABLE 6**

| | GSH (µmoles/mg protein) | MDA (moles/g tissue) |
|---|---|---|
| Control | 12.7911.22 | 6.9110.35 |
| Example 50 mg/kg | 13.1010.98 | 5.4810.17* |
| Example 200 mg/kg | 15.7711.21* | 4.3410.12* |

| | | |
|---|---|---|
| Comparative control with statistical significance *: *p<0.05* | | |

As shown in FIGS. 7a and 7b, the level of glutathione, which exhibits non-enzymatic antioxidant activity in liver tissues, was increased in the Example extract administration group, compared to the control group. In particular, glutathione increased by about 1.02 times and about 1.23 times in the 50 mg/kg- and 200 mg/kg-administered groups, respectively, compared to the control group. In addition, the level of malondialdehyde, which is a mediator of the oxidation reaction, showed a significant decrease in all of the Example extract-administered groups, compared to the control group. The data demonstrated that the Example extract had excellent effects on antioxidant enzyme activity in liver tissues.

### Industrial Applicability

The present disclosure relates to a composition for prevention or treatment of or recovery from fatigue.

## Claims

1. A food composition for prevention of or recovery from fatigue, the composition comprising an extract from at least one selected from the group consisting of Cynanchi Wilfordii Radix, Phlomis umbrosa TURCZ., and angelica.

2. The food composition of claim 1, wherein the fatigue is central nervous system fatigue, nerve-muscular joint fatigue, or peripheral fatigue of the limbs.

3. The food composition of claim 1, wherein the fatigue is accompanied by decreased exercise performance, chronic fatigue, sleep disorder, mental concentration disorder, muscle pain, arthralgia, headache, sore throat, or lymphadenitis.

4. The food composition of claim 1, wherein the extract is a crude extract obtained by extraction with at least one solvent selected from the group consisting of water and a straight or branched alcohol of 1 to 4 carbon atoms.

5. A pharmaceutical composition for prevention or treatment of fatigue, the composition comprising an extract from at least one selected from the group consisting of Cynanchi Wilfordii Radix, Phlomis umbrosa TURCZ., and angelica.

6. The pharmaceutical composition of claim 5, wherein the fatigue is central nervous system fatigue, nerve-muscular joint fatigue, or peripheral fatigue of the limbs.

7. The pharmaceutical composition of claim 5, wherein the fatigue is accompanied by decreased exercise performance, chronic fatigue, sleep disorder, mental concentration disorder, muscle pain, arthralgia, headache, sore throat, or lymphadenitis.

8. The pharmaceutical composition of claim 5, wherein the extract is a crude extract obtained by extraction with at least one solvent selected from the group consisting of water and a straight or branched alcohol of 1 to 4 carbon atoms.
